# DEMANDE DE BREVET EUROPEEN

(11) **EP 3 753 477 A1**
(43) Date de publication de la demande: **23.12.2020**
(21) Numéro de dépôt: 20179517.6
(22) Date de dépôt: 11.06.2020
(51) Int. Cl.: A61B 5/00, A61B 5/08, A61B 5/091

(54) **SYSTÈME D'ALERTE SUR TENDANCE EN VENTILATION NON INVASIVE**

(30) Priorité: 18.06.2019 FR 1906501
(71) Demandeur: Pharma Dom, 94250 Gentilly (FR); Alehos Development, 94250 Gentilly (FR); Vitalaire, 75007 Paris (FR); L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Inventeur: TEXEREAU, Joëlle, 75007 Paris (FR); TERRIEN, Didier, 49070 Beaucouze (FR); MUNTANER, Bertrand, 94250 Gentilly (FR)
(74) Mandataire: Air Liquide

(57) **Abrégé**

L'invention concerne un système de télésurveillance (1, 10, 20) d'un ou plusieurs paramètres de ventilation non invasive (VNI) d'au moins un patient sous ventilation non invasive (VNI) traité à son domicile comprenant des moyens informatiques de traitement de données (10) comprenant des moyens de réception de données configurés pour recevoir ledit au moins un paramètre ventilatoire provenant d'un ventilateur médical (1) et transmis par des moyens de transmission de données (2, 3, 4) recueillant ledit au moins un paramètre ventilatoire dudit ventilateur médical, et au moins un processeur mettant en œuvre au moins un algorithme pour traiter ledit au moins un paramètre ventilatoire et obtenir au moins une valeur traitée dudit au moins un paramètre ventilatoire ; comparer ladite au moins une valeur traitée avec au moins une valeur-seuil mémorisée, et déclencher une alerte lorsque ladite au moins une valeur traitée excède ladite au moins une valeur-seuil mémorisée ; et des moyens d'alerte (20) configurés pour avertir ou informer un utilisateur (21) en réponse à un déclenchement d'alerte par ledit au moins un processeur des moyens informatiques de traitement de données (10). Préférentiellement, il comprend en outre un ventilateur médical (1) comprenant des moyens de mesure de paramètres configurés pour mesurer au moins un paramètre ventilatoire, en particulier la fréquence respiratoire ou la durée d'utilisation.

## Description

L'invention concerne un système de télésurveillance, i.e. un dispositif médical de suivi à distance, d'un (ou plusieurs) patient sous ventilation non invasive (VNI) traité à son domicile comprenant des moyens informatiques de traitement de données pour détecter à distance une ventilation opérée par un ventilateur médical, qui n'est pas ou pas assez efficace ou un état clinique qui se dégrade chez le patient et des moyens d'alerte configurés pour informer et/ou alerter un utilisateur en cas d'une telle détection, en particulier un prestataire de santé à domicile (PSAD).

L'insuffisance respiratoire chronique est définie par l'incapacité permanente du système respiratoire à oxygéner correctement le sang au repos. Elle constitue un handicap sévère limitant la vie quotidienne des patients et impactant leur espérance de vie.

Les patients atteints d'insuffisance respiratoire chronique grave nécessitent parfois la mise en route d'une ventilation non invasive (VNI) à domicile, c'est-à-dire hors milieu hospitalier ou analogue, et sur des durées longues, typiquement plusieurs mois ou années. Durant une VNI, on apporte un support mécanique à l'action des muscles respiratoires en insufflant un gaz respiratoire dans les voies aériennes du patient en utilisant un appareil d'assistance respiratoire, aussi appelé ventilateur médical, délivrant le gaz respiratoire sous pression positive, typiquement de l'air, éventuellement enrichi en oxygène.

La VNI est un traitement que les patients utilisent la nuit et pendant quelques heures durant la journée ou bien en permanence, en fonction de la gravité de leur insuffisance respiratoire. Les principales pathologies pouvant conduire à l'insuffisance respiratoire chronique grave sont la bronchopneumopathie chronique obstructive (BPCO), le syndrome obésité-hypoventilation (SOH), les maladies respiratoires restrictives comme la cyphoscoliose ou la fibrose pulmonaire, les maladies neuromusculaires comme la sclérose latérale amyotrophique (SLA) ou la myopathie de Duchenne. En France, environ 60 000 patients sont traités par ventilation mécanique à domicile (source: ameli.fr).

Le suivi des patients insuffisants respiratoires traités par ventilation non invasive à domicile nécessite des visites régulières d'intervenants (technicien et/ou infirmier) au domicile du patient, tous les deux à quatre mois, notamment pour vérifier l'efficacité clinique, l'observance et la survenue d'éventuels effets indésirables et effectuer les relevés détaillés du ventilateur, à destination du médecin prescripteur. Ce suivi permet de faire des actions visant au renforcement de l'observance, à la limitation des effets indésirables (comme les fuites au masque) et d'opérer des réglages ou ajustements des paramètres du ventilateur pour améliorer l'efficacité de la ventilation. En effet, les réglages cibles du ventilateur, ainsi que les paramètres à surveiller médicalement dépendent de la ou des pathologies sous-jacentes et sont propres à chaque patient.

Lors de ces visites, les données qui sont mémorisées par le ventilateur sont déchargées et récupérées par le technicien de manière à lui permettre et par ailleurs au médecin prescripteur, d'analyser le suivi du traitement par le patient, sa tolérance et donc son efficacité pendant les mois écoulés et permettre éventuellement des réglages des paramètres du ventilateur ou ajustements (changement de masque...).

Cependant, malgré ces suivis réguliers, beaucoup (i.e. > 30%) de patients sous VNI à domicile sont traités de manière non totalement efficace du fait de réglages mal adaptés ou d'autres raisons, comme une lassitude ou un découragement du patient entraînant une baisse d'observance de son traitement, la survenue d'un problème technique (e.g. ventilateur, masque...) générant des fuites ou une asynchronie entre le patient et le ventilateur...

Les variations des paramètres physiologiques mesurés, comme la fréquence respiratoire, pourraient également contribuer à détecter rapidement, et à distance, la survenue d'événements aigus respiratoires chez certains patients, comme des exacerbations chez des patients atteints de BPCO, permettant un diagnostic plus précoce, et donc potentiellement à un stade de sévérité moindre, de l'événement aigu.

Or, on comprend que cette situation n'est pas idéale et qu'il existe un besoin de pouvoir améliorer le suivi du traitement des patients sous VNI à domicile.

FR-A-3021872 enseigne un procédé de détection d'une aggravation d'un état cardiopulmonaire d'un patient relié à un ventilateur et à une source d'oxygène, lequel est basé sur la fréquence respiratoire, le nombre de cycles respiratoires et la durée d'utilisation de l'appareil qui sont déterminés sur plusieurs fenêtres temporelles quotidiennes stockées sur une mémoire du ventilateur médical. Une alerte est émise en cas de variation significative d'un ou plusieurs de ces paramètres.

Au vu de cela, le problème qui se pose est de pouvoir détecter rapidement une VNI non efficace, insuffisante ou un état de santé qui se dégrade chez un patient sous VNI traité à son domicile avec un ventilateur médical, afin d'intervenir rapidement et éviter un impact sur l'état de santé dudit patient.

La solution de l'invention concerne un système de télésurveillance, i.e. un dispositif médical de suivi à distance, d'un ou plusieurs paramètres de ventilation non invasive (VNI) d'au moins un patient traité à son domicile au moyen d'un ventilateur médical, comprenant :
- un ventilateur médical comprenant :
   - des moyens de mesure de paramètres configurés pour déterminer au moins un paramètre ventilatoire, i.e. un paramètre de ventilation non invasive (VNI), choisis parmi la fréquence respiratoire (FR) et la durée d'utilisation quotidienne du ventilateur médical, et
   - des moyens de transmission de données configurés pour transmettre à distance au moins ledit paramètre ventilatoire,
- des moyens informatiques de traitement de données comprenant :
   - des moyens de réception de données configurés pour recevoir ledit au moins un paramètre ventilatoire provenant dudit ventilateur médical et transmis par des moyens de transmission de données recueillant ledit au moins un paramètre ventilatoire dudit ventilateur médical, et
   - au moins un processeur mettant en oeuvre au moins un algorithme pour :
      i) traiter ledit au moins un paramètre ventilatoire et obtenir au moins une valeur traitée dudit au moins un paramètre ventilatoire,
      ii) comparer ladite au moins une valeur traitée avec au moins une valeur-seuil mémorisée, et
      iii) déclencher une alerte lorsque ladite au moins une valeur traitée excède ladite au moins une valeur-seuil mémorisée,
- et des moyens d'alerte configurés pour avertir ou informer un utilisateur en réponse à un déclenchement d'alerte par ledit au moins un processeur des moyens informatiques de traitement de données,
caractérisé en ce que :
a) l'algorithme du microprocesseur est configuré pour :
   i) rechercher un changement de la durée d'utilisation quotidienne du ventilateur, c'est-à-dire de la durée de traitement du patient, à partir d'un coefficient interquartile relatif déterminé à partir de plusieurs mesures de durée d'utilisation quotidienne opérées pendant une fenêtre temporelle de référence d'au moins 5 jours consécutifs, de préférence de 5 à 10 jours consécutifs, une télétransmission de ces mesures d'utilisation quotidienne étant effectuée chaque jour de ladite fenêtre temporelle de référence, chaque mesure d'utilisation quotidienne télétransmise correspondant à la somme des périodes d'utilisation du ventilateur par le patient pendant une période de temps égale à 1 jour (i.e. les mesures d'utilisation quotidienne télétransmises couvrent des périodes de 24 heures successives et disjointes), et
   ii) déclencher une alerte de variation du profil d'utilisation à la hausse lorsque le coefficient interquartile relatif déterminé excède 20% et que la durée d'utilisation quotidienne du ventilateur a augmenté d'au moins 120 minutes pendant ladite fenêtre temporelle de référence d'au moins 5 jours consécutifs, de préférence de 5 à 10 jours consécutifs,
      ou
b) l'algorithme du microprocesseur est configuré pour :
   i) rechercher un changement de la fréquence respiratoire (FR) à partir du taux de variation de plusieurs écarts-types successifs déterminés sur une fenêtre temporelle glissante de 10 jours, ladite fenêtre temporelle glissant de 1 jour pour chaque calcul d'écart-type, à partir de mesures de fréquence respiratoire (FR) télétransmises chaque jour par le ventilateur, chaque mesure de fréquence respiratoire (FR) télétransmise correspondant à la moyenne ou à la médiane des fréquences respiratoires du patient mesurées par le ventilateur pendant une période de temps égale à 1 jour (i.e. les mesures de fréquence respiratoire télétransmises couvrent des périodes de 24 heures successives et disjointes), et
   ii) déclencher une alerte de variation de la fréquence respiratoire (FR) à la hausse ou à la baisse lorsque le taux de variation déterminé excède une valeur-seuil fixée à 5%, durant plusieurs jours consécutifs, typiquement 2 jours consécutifs.

Selon le mode de réalisation considéré, le système de surveillance de l'invention peut comprendre l'une ou plusieurs des caractéristiques suivantes :
- l'algorithme du microprocesseur est configuré pour opérer une recherche du changement de la fréquence respiratoire (FR) à partir du taux de variation d'au moins 3 écarts-types successifs, typiquement 3 écarts-types successifs sont nécessaires pour déterminer une augmentation du taux de variation sur 2 jours successifs.
- l'algorithme du microprocesseur est configuré pour rechercher un changement de la durée d'utilisation quotidienne du ventilateur à partir d'un coefficient interquartile relatif déterminé à partir de plusieurs mesures de durée d'utilisation quotidienne opérées pendant une fenêtre temporelle de référence de 5 à 10 jours consécutifs.
- l'algorithme du microprocesseur est configuré pour déclencher une alerte de variation de la fréquence respiratoire (FR) à la hausse ou à la baisse lorsque le taux de variation déterminé excède une valeur-seuil fixée à 5%, durant 2 jours consécutifs.
- la fréquence respiratoire (FR) est une fréquence respiratoire médiane ou moyenne selon le ventilateur médical considéré.
- l'algorithme du microprocesseur est configuré pour exclure, avant tout calcul, les mesures télétransmises indiquant une durée d'utilisation journalière inférieure à 90 minutes pour la détermination d'un changement de fréquence respiratoire (FR) ou inférieure à 360 minutes pour la détermination d'un changement de durée d'utilisation quotidienne du ventilateur.
- l'algorithme du microprocesseur est configuré pour opérer une détermination d'un changement de fréquence respiratoire (FR) ou de durée d'utilisation quotidienne du ventilateur à partir de données obtenues sur au moins 5 jours.
- les moyens de mesure de paramètres du ventilateur médical sont configurés pour déterminer ou mesurer au moins un paramètre ventilatoire au moins 1 fois par jour, c'est-à-dire que le ou les paramètres sont déterminés et/ou mesurés au moins 1 fois par jour.
- le ventilateur médical comprend en outre des moyens de transmission de données, par exemple un modem ou analogue, configurés pour transmettre à distance au moins ledit paramètre ventilatoire.
- les moyens de transmission de données sont soit intégrés au ventilateur, soit faire partie d'un module externe coopérant avec le ventilateur, par exemple venant se fixer ou raccorder audit ventilateur médical.
- l'alerte déclenchée par les moyens d'alerte afin d'avertir ou d'informer l'utilisateur, comprend une notification préalable, par exemple un ou des caractères (lettres, chiffres ou autres), notamment un message en format texte, un code couleur, une icône, un barre-graphe, ou autre et/ou leurs combinaisons, par exemple un barre-graphe de plusieurs couleurs.
- le ou les paramètres ventilatoires provenant du ventilateur médical comprennent en outre un ou plusieurs paramètres primaires choisis parmi les fuites et un événement respiratoire anormal, tel qu'apnées, hypopnées ou autres.
- le ou les paramètres ventilatoires provenant du ventilateur médical comprennent un ou plusieurs paramètres secondaires choisis parmi le volume courant (Vt), la proportion (i.e. %) d'inspirations spontanées (par rapport au nombre total de cycles inspiratoires, i.e. déclenchés par le ventilateur et le patient), et la ventilation minute.
- le ou les paramètres provenant du ventilateur médical comprennent en outre un ou plusieurs paramètres de fonctionnement, notamment une information relative à une absence de transmission de données par le ventilateur médical. Ceci permet d'avertir l'utilisateur (e.g. PSAD) d'un défaut ou dysfonctionnement du ventilateur affectant la bonne transmission des données, i.e. paramètres.
- l'algorithme est par ailleurs configuré pour contrôler la pertinence des données utilisées dans le calcul. Les données non pertinentes sont retirées et ne sont pas utilisées pour opérer le ou les calculs. Si l'algorithme dispose de moins de 5 données valides par exemple, le calcul de l'indicateur de variation n'a pas lieu. Par exemple, une donnée est considérée comme non valide en cas de trop fortes fuites. Pour le calcul de l'indicateur de variation de la fréquence respiratoire, une durée d'utilisation trop faible est aussi un critère de non pertinence, typiquement moins de 90 min par jour.
- le ventilateur médical fournit de l'air ou un mélange air/oxygène à une pression supérieure à la pression atmosphérique (i.e. 1 bar absolu).
- les moyens de transmission de données comprennent un modem ou analogue.
- les moyens informatiques de traitement de données comprennent au moins un serveur informatique.
- le processeur est un microprocesseur ou analogue.
- le processeur est agencé sur une carte électronique ou analogue.
- il comprend des moyens de mémorisation de données configurés pour mémoriser le ou les paramètres ventilatoires provenant du ventilateur médical et/ou au moins une valeur-seuil.
- les moyens de mémorisation de données sont configurés pour mémoriser au moins une valeur-seuil d'alerte de fréquence respiratoire et/ou de durée d'utilisation quotidienne.
- les moyens d'alerte comprennent un afficheur, c'est-à-dire un écran de visualisation, configuré pour afficher une alerte visuelle.
- les moyens d'alerte comprennent un ordinateur, une tablette numérique ou un téléphone intelligent (i.e. smartphone).

Dans le cadre de la présente invention :
- la durée d'utilisation de la ventilation non invasive est la durée pendant laquelle le patient a utilisé le ventilateur pendant une période de temps donnée, typiquement une journée (i.e. 24h). Plusieurs durées d'utilisation successives permettent de déterminer une tendance à la hausse ou à la baisse d'utilisation du ventilateur par le patient et/ou une fragmentation de cette utilisation.
- la fréquence respiratoire (FR) est le nombre de cycles respiratoires, i.e. phases d'inspiration et d'expiration, (i.e. médian ou moyen) par unité de temps, mesurés chez un patient, par exemple elle est de 12 à 20 cycles par minute (cycles/min) chez un adulte sain mais qui peut augmenter en cas de troubles respiratoires ou diminuer pendant le sommeil.
- les fuites sont les fuites ou échappements d'air autour du masque, limitant l'efficacité de la ventilation et source d'inconfort pour le patient. Les fuites sont estimées par le ventilateur à partir par exemple des débits de gaz mesurés.
- la proportion (%) d'inspirations spontanées correspond au pourcentage des cycles respiratoires qui sont initiés par le patient, et non par le ventilateur, rapporté au nombre total de cycles.
- le nombre d'événements respiratoires (i.e. apnées, hypopnées) par heure de traitement correspond au nombre d'arrêts et/ou de diminutions du flux d'air pendant une période de temps donnée, par exemple supérieure ou égale à 10 secondes, aussi appelé index d'apnées-hypopnées.
- le volume courant (Vt) est le volume d'air entrant dans les poumons du patient à chaque cycle respiratoire, par exemple un volume d'air d'environ 0,5 L.
- la ventilation minute correspond au produit Vt.FR.

L'invention porte aussi sur une utilisation d'un système selon l'invention pour opérer le suivi d'un patient souffrant d'une insuffisance respiratoire chronique grave, notamment une bronchopneumopathie chronique obstructive (BPCO), un syndrome obésité-hypoventilation (SOH), ou une maladie neuromusculaire, telle la sclérose latérale amyotrophique ou la myopathie de Duchenne.

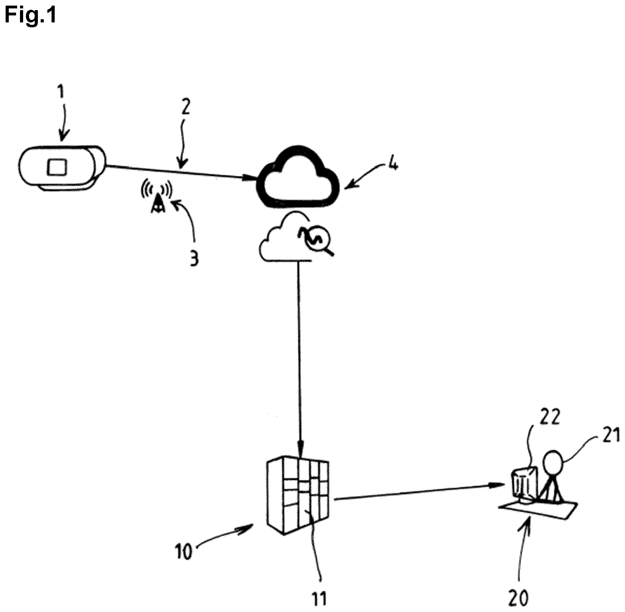

L'invention va maintenant être mieux comprise grâce à la description détaillée suivante, faite à titre illustratif mais non limitatif, en référence à la Fig. 1 qui schématise un système de surveillance d'un patient sous ventilation non invasive (VNI) selon l'invention.

La Fig. 1 schématise un système de surveillance 1, 10, 20 d'un patient sous ventilation non invasive (VNI) traité à son domicile pour une insuffisance respiratoire chronique grave, notamment une bronchopneumopathie chronique obstructive (BPCO), un syndrome obésité-hypoventilation (SOH) ou une maladie neuromusculaire, telle la sclérose latérale amyotrophique ou la myopathie de Duchenne.

Dans le cadre de son traitement, le patient reçoit un gaz d'assistance respiratoire sous pression (i.e. > 1 bar abs), tel de l'air ou un mélange air/O₂, délivré par un ventilateur médical 1, c'est-à-dire un appareil d'assistance respiratoire. De manière classique, le gaz est acheminé jusqu'aux voies aériennes du patient par un tuyau flexible relié fluidiquement au ventilateur 1 et équipé d'une interface respiratoire patient, tel un masque ou analogue (non montré).

Par ailleurs, le ventilateur médical 1 est alimenté électriquement par le réseau électrique (110/220V) ou par une ou des batteries internes, notamment rechargeables, ou analogue. Le ventilateur médical 1 comprend des moyens de mesure de paramètres configurés pour suivre un ou des paramètres ventilatoires, en particulier la fréquence respiratoire (exprimée en cycles/min) et la durée d'utilisation quotidienne (exprimée en minutes par exemple) du ventilateur par le patient qui reflète la durée pendant laquelle le patient a inhalé du gaz respiratoire, c'est-à-dire a suivi son traitement.

Sont également prévus des moyens de télétransmission de données configurés pour transmettre à distance au moins les paramètres ventilatoires fournis par les moyens de mesure de paramètres, par exemple ils comprennent un modem de type GSM ou autre et une antenne émettrice. Les paramètres transmis transitent préférentiellement via le réseau internet 2 ou un autre réseau 3, tels les réseaux GSM, Lora ou Sigfox, et/ou peuvent être traitées dans l'espace informatique virtuel 4 ou « cloud ».

Le traitement des paramètres est opéré via des moyens informatiques de traitement de données 10 comprenant des moyens de réception de données, configurés pour recevoir les paramètres ventilatoires télétransmis par les moyens de transmission de données du ventilateur 1, ainsi qu'un (ou plusieurs) processeur mettant en œuvre un ou des algorithmes.

De préférence, chaque jour, le système de l'invention enregistre et traite les dernières données transmises par le ventilateur 1, en particulier la durée d'utilisation quotidienne et la fréquence respiratoire (FR) et ce, pendant plusieurs jours consécutifs, i.e. au moins 5 jours, de préférence entre 5 et 10 jours.

Pour ce faire, le système 1 de l'invention comprend aussi des moyens de mémorisation de données configurés pour mémoriser le ou les paramètres ventilatoires provenant du ventilateur médical 1, par exemple une carte-mémoire de stockage de donnée. Chaque jour, les dernières données, c'est-à-dire le ou les paramètres ventilatoires les plus récents non-encore envoyés, transmises par le ventilateur 1 y sont mémorisées.

Par ailleurs, on utilise au moins un algorithme mis en oeuvre par un microprocesseur pour traiter le ou les paramètres ventilatoires mesurés ou déterminés et comparer le(s) résultat(s) de ce traitement avec une ou plusieurs valeurs-seuils, et déclencher une alerte visuelle et/ou sonore, lorsque ce ou ces paramètres ventilatoires excèdent de la ou des valeurs-seuils considérées.

A titre d'exemple, on peut utiliser la fréquence respiratoire (FR) et la durée d'utilisation quotidienne du ventilateur par le patient comme paramètres qui sont traités pour obtenir un ou des indicateurs, lesquels sont ensuite comparés à une ou plusieurs valeurs-seuils prédéfinies, pour déclencher éventuellement une alerte visant à informer l'utilisateur, tel un PSAD. Selon l'invention, les indicateurs peuvent être un coefficient interquartile relatif et/ou une augmentation du taux de variation ou proportion de variation entre des écarts-types.

Ainsi, l'indicateur de changement de la durée d'utilisation quotidienne est un coefficient interquartile relatif déterminé sur les n dernières mesures (n>1), par exemple n=8, c'est-à-dire sur une durée d'au moins 5 jours, de préférence de 5 à 10 jours consécutifs (i.e. 5 < n <10). La valeur-seuil du coefficient interquartile relatif est fixée à 20% pour déclencher une alerte de variation du profil d'utilisation à la hausse, après avoir exclu les jours où la durée d'utilisation journalière est inférieure à 360 minutes. Un algorithme du microprocesseur est configuré pour rechercher une évolution à la hausse, c'est-à-dire qu'entre le premier jour de durée d'utilisation journalière supérieure à 360 minutes et le dernier jour, la donnée doit augmenter d'au moins 120 minutes. Grâce à ce mode de calcul, l'algorithme est peu sensible aux perturbations générées par les valeurs extrêmes. L'indicateur étant invariant par changement d'échelle, il n'est pas nécessaire d'ajuster le seuil en fonction du patient.

Par ailleurs, l'indicateur de changement de la fréquence respiratoire (FR) est une augmentation du taux de variation entre l'écart-type déterminé sur les n (n>1) dernières mesures reçues (par exemple J-10; J-1) et l'écart-type déterminé sur les n avant-dernières mesures (par exemple J-11; J-2) et les n avant-avant-dernières mesures (par exemple J-12; J-3). La valeur-seuil d'augmentation du taux de variation est fixée à quelques %, par exemple de l'ordre de 5%, durant plusieurs jours consécutifs, par exemple 2 jours, pour déclencher une alerte de variation de la fréquence respiratoire. Un algorithme du microprocesseur est configuré pour pouvoir rechercher une évolution de la fréquence respiratoire (FR) à la hausse ou à la baisse.

Les valeurs-seuils fixées pour le déclenchement d'une ou des alertes ont un sens physique clair. Les seuls cas où l'algorithme pourrait ne pas être adapté, correspondent aux valeurs médianes nulles ou très proche de 0. Or, la fréquence respiratoire chez un patient n'étant jamais (quasi)nulle, il est toujours possible de déterminer un écart interquartile chez un patient donné, par exemple sur plusieurs jours, et de les comparer avec des valeurs-seuils correspondantes.

Quant à la durée d'utilisation quotidienne, celle-ci peut être (quasi)nulle, en particulier si le patient n'utilise pas son traitement pendant plusieurs jours, par exemple pendant plus de 3 jours sur les 8 jours retenus. Toutefois, dans ce cas, l'algorithme est configuré pour contrôler d'abord cette situation (i.e. durée durée d'utilisation quotidienne nulle) et pour alerter immédiatement, le cas échéant.

D'une façon générale, le coefficient interquartile relatif ou l'écart-type utilisés par l'algorithme dans le cadre de la présente invention sont notamment expliqués et illustrés par les sites internet suivants:
http://grastand.script.univ-paris-diderot.fr/STAT98/stat98 4/stat98 4.htm http://margaux.ipt.univ-paris8.fr/vgodard/enseigne/statisti/memostat/mem41sta.htm

Dans le cadre de la présente invention, l'algorithme est préférentiellement hébergé sur un (ou des) serveur central 11 et permet de traiter à distance les mesures (i.e. paramètres mesurés), en particulier la fréquence respiratoire (FR) et/ou la durée d'utilisation opérées sur plusieurs jours, et télé transmises par le ventilateur 1 communicant situé au domicile du patient.

Par ailleurs, on prévoit aussi des moyens d'alerte 20 configurés pour alerter un utilisateur 21, tel un prestataire de santé à domicile (PSAD), en réponse à un déclenchement d'alerte par le processeur des moyens informatiques de traitement de données 10.

En d'autres termes, l'algorithme calcule pour chaque patient, un ou des indicateurs de modification des paramètres par rapport aux paramètres du patient, en particulier fréquence respiratoire (FR) et durée d'utilisation quotidienne, évalués sur une période de référence, par exemple sur plusieurs jours, à savoir au moins 5 jours, typiquement de 5 à 10 jours consécutifs, et si l'un de ces indicateurs dépasse une valeur-seuil préprogrammée et/ou mémorisée, une notification d'alerte est déclenchée pour ce patient de manière à informer et/ou avertir l'utilisateur 21, tel un prestataire de soins à domicile (PSAD), qu'il peut être nécessaire d'intervenir au domicile du patient considéré.

La notification d'alerte déclenchée par les moyens d'alerte peut prendre la forme d'une alerte visuelle, telle une notification, s'affichant sur un écran de visualisation 22, tel un afficheur d'ordinateur, de téléphone ou de tablette numérique. La notification peut comprendre par exemple un ou des caractères (i.e. lettres, chiffres ou autres symboles), notamment un message en format texte, mais aussi un code couleur, une icône, un barre-graphe ou tout autre représentation graphique, et/ou leurs combinaisons, par exemple un barre-graphe de plusieurs couleurs, par exemple vert, jaune, orange et rouge en fonction de la gravité de l'alerte.

D'une façon générale, le système de l'invention permet de détecter à distance, une VNI non efficace, insuffisante ou qui se dégrade chez un patient donné, et d'alerter un utilisateur 21, tel un PSAD ou une équipe médicale, pour lui permettre d'intervenir rapidement au domicile du patient de manière à éviter ou minimiser tout impact négatif sur l'état de santé dudit patient. Le système de l'invention permet donc de détecter rapidement, efficacement et à distance un ou des éléments/indicateurs représentatifs d'une ventilation de type VNI qui n'est pas ou pas assez efficace ou qui se dégrade, et ainsi de mettre en place des actions correctives techniques ou d'informer les professionnels de santé sur une baisse de l'observance ou une variation trop importante de la fréquence respiratoire du patient, ce qui peut être un signe précurseur de dégradation de l'état de santé de ce patient.

L'invention est destinée à une utilisation à des fins médicales car elle peut permettre une aide au diagnostic (e.g. détection d'une dégradation de l'état de santé) ou au traitement. Elle donne un résultat propre au bénéfice d'un seul patient. Elle comprend une analyse opérée sur les données entrantes (i.e. signaux physiologiques et/ou techniques) propres à un patient afin de fournir une information nouvelle et est dotée de fonctions d'alertes à finalité médicale. A ce titre, elle répond à des critères de dispositif médical de classe IIA.

D'une façon générale, le système de l'invention est compatible avec tous les ventilateurs 1 communicants et est en outre capable de censurer les paramètres mesurés pendant des périodes d'observance trop faibles afin que la valeur soit pertinente.

## Revendications

1. Système de télésurveillance (1, 10, 20) d'un ou plusieurs paramètres de ventilation non invasive (VNI) d'au moins un patient sous ventilation non invasive (VNI) traité à son domicile au moyen d'un ventilateur médical comprenant :
- un ventilateur médical comprenant :
• des moyens de mesure de paramètres configurés pour déterminer au moins un paramètre ventilatoire choisi parmi la fréquence respiratoire (FR) et la durée d'utilisation quotidienne du ventilateur médical, et
• des moyens de transmission de données configurés pour transmettre à distance au moins ledit paramètre ventilatoire,
- des moyens informatiques de traitement de données (10) comprenant :
• des moyens de réception de données configurés pour recevoir ledit au moins un paramètre ventilatoire provenant dudit ventilateur médical (1) et transmis par des moyens de transmission de données (2, 3, 4) recueillant ledit au moins un paramètre ventilatoire dudit ventilateur médical (1), et
• au moins un processeur mettant en œuvre au moins un algorithme pour :
a) traiter ledit au moins un paramètre ventilatoire et obtenir au moins une valeur traitée dudit au moins un paramètre ventilatoire,
b) comparer ladite au moins une valeur traitée avec au moins une valeur-seuil mémorisée, et
c) déclencher une alerte lorsque ladite au moins une valeur traitée excède ladite au moins une valeur-seuil mémorisée,
- et des moyens d'alerte (20) configurés pour avertir ou informer un utilisateur (21) en réponse à un déclenchement d'alerte par ledit au moins un processeur des moyens informatiques de traitement de données (10),
**caractérisé en ce que** :
i) l'algorithme du microprocesseur est configuré pour :
A) rechercher un changement de la durée d'utilisation quotidienne du ventilateur à partir d'un coefficient interquartile relatif déterminé à partir de plusieurs mesures de durée d'utilisation quotidienne opérées pendant une fenêtre temporelle de référence d'au moins 5 jours consécutifs, une télétransmission de ces mesures d'utilisation quotidienne étant effectuées chaque jour de ladite fenêtre temporelle de référence, chaque mesure d'utilisation quotidienne télétransmise correspondant à la somme des périodes d'utilisation du ventilateur par le patient pendant une période de temps égale à 1 jour, et
B) déclencher une alerte de variation du profil d'utilisation à la hausse lorsque le coefficient interquartile relatif déterminé excède 20% et que la durée d'utilisation quotidienne du ventilateur a augmenté d'au moins 120 minutes pendant ladite fenêtre temporelle de référence d'au moins 5 jours consécutifs,
et/ou
ii) l'algorithme du microprocesseur est configuré pour :
A) rechercher un changement de la fréquence respiratoire (FR) à partir du taux de variation de plusieurs écarts-types successifs déterminés sur une fenêtre temporelle glissante de 10 jours, ladite fenêtre temporelle glissant de 1 jour pour chaque calcul d'écart-type, à partir de mesures de fréquence respiratoire (FR) télétransmises chaque jour, chaque mesure de fréquence respiratoire (FR) télétransmise correspondant à la moyenne ou à la médiane des fréquences respiratoires du patient mesurées par le ventilateur pendant une période de temps égale à 1 jour, et
B) déclencher une alerte de variation de la fréquence respiratoire (FR) à la hausse ou à la baisse lorsque le taux de variation déterminé excède une valeur-seuil fixée à 5%, durant plusieurs jours consécutifs.

2. Système selon la revendication précédente, **caractérisé en ce que** le ventilateur médical (1) comprend des moyens de transmission de données configurés pour transmettre à distance au moins ledit paramètre ventilatoire.

3. Système selon l'une des revendications précédentes, **caractérisé en ce que** les moyens d'alerte (20) comprennent un écran de visualisation (22) configuré pour afficher une alerte visuelle.

4. Système selon l'une des revendications précédentes, **caractérisé en ce que** les moyens d'alerte (20) comprennent un ordinateur, une tablette numérique ou un téléphone intelligent.

5. Système selon l'une des revendications précédentes, **caractérisé en ce que** les moyens informatiques de traitement de données (10) comprennent au moins un serveur informatique (11).

6. Système selon l'une des revendications précédentes, **caractérisé en ce qu'**il comprend des moyens de mémorisation de données configurés pour mémoriser le ou les paramètres ventilatoires provenant du ventilateur médical (1) et/ou au moins une valeur-seuil.

7. Système selon l'une des revendications précédentes, **caractérisé en ce que** l'algorithme du microprocesseur est configuré pour opérer une recherche du changement de la fréquence respiratoire (FR) à partir du taux de variation d'au moins 3 écarts-types successifs.

8. Système selon l'une des revendications précédentes, **caractérisé en ce que** l'algorithme du microprocesseur est configuré pour rechercher un changement de la durée d'utilisation quotidienne du ventilateur à partir d'un coefficient interquartile relatif déterminé à partir de plusieurs mesures de durée d'utilisation quotidienne opérées pendant une fenêtre temporelle de référence de 5 à 10 jours consécutifs.

9. Système selon l'une des revendications précédentes, **caractérisé en ce que** l'algorithme du microprocesseur est configuré pour déclencher une alerte de variation de la fréquence respiratoire (FR) à la hausse ou à la baisse lorsque le taux de variation déterminé excède une valeur-seuil fixée à 5%, durant 2 jours consécutifs.

10. Système selon l'une des revendications précédentes, **caractérisé en ce que** l'algorithme du microprocesseur est configuré pour exclure, avant tout calcul, les mesures télétransmises indiquant une durée d'utilisation journalière inférieure à 90 minutes pour la détermination d'un changement de fréquence respiratoire (FR) ou inférieure à 360 minutes pour la détermination d'un changement de durée d'utilisation quotidienne du ventilateur (1).

11. Système selon l'une des revendications précédentes, **caractérisé en ce que** l'algorithme du microprocesseur est configuré pour opérer une détermination d'un changement de fréquence respiratoire (FR) ou de durée d'utilisation quotidienne du ventilateur (1) à partir de données obtenues sur au moins 5 jours.

12. Système selon l'une des revendications précédentes, **caractérisé en ce que** les moyens de transmission de données sont intégrés au ventilateur ou font partie d'un module externe coopérant avec le ventilateur (1).

13. Système selon l'une des revendications précédentes, **caractérisé en ce que** les moyens de mesure de paramètres du ventilateur médical (1) sont configurés pour déterminer ou mesurer au moins un paramètre ventilatoire au moins 1 fois par jour.
